# DEMANDE DE BREVET EUROPEEN

(11) **EP 0 955 011 A1**
(43) Date de publication de la demande: **10.11.1999**
(21) Numéro de dépôt: 98440086.1
(22) Date de dépôt: 06.05.1998
(51) Int. Cl.: A61B 17/064

(54) **Agrafe chirurgicale utilisée dans le domaine de la chururgie orthopedique**

(71) Demandeur: EOS Sarl, 38200 Vienne (FR)
(72) Inventeur: Granger, Andre, 88650 Anould (FR)
(74) Mandataire: Arbousse-Bastide, Jean-Claude Philippe

(57) **Abrégé**

Agrafe chirurgicale utilisée dans le domaine de la chirurgie orthopédique.

Elle comporte deux jambes (1) sensiblement parallèles reliées par une partie transversale (3) présentant deux branches (4) et formant un oeil (5) dont la déformation en écartement, dans le sens perpendiculaire à l'axe de la partie transversale (3), permet le rapprochement des jambes (1).

Les branches (4) comportent plusieurs zones de flexion (31, 41) de manière à permettre la déformation de l'oeil (5) en exerçant une pression, dans le sens axial de la partie transversale (3), sur les extrémités (22) des jambes (2) qui émergent de la matière osseuse.

## Description

La présente invention a pour objet une agrafe chirurgicale utilisée dans le domaine de la chirurgie orthopédique.

Une agrafe chirurgicale est essentiellement destinée à la consolidation et au rapprochement de deux os ou de deux fragments d'os, à la suite d'une fracture par exemple, et elle comporte généralement deux jambes sensiblement parallèles reliées par une partie transversale. Les deux jambes sont destinées à être introduites dans la matière osseuse de part et d'autre du trait de fracture, après que l'on ait réalisé deux perçages d'un entraxe correspondant à celui desdites jambes.

L'inconvénient de ces agrafes réside dans le fait qu'elles ne permettent que le maintien des deux fragments d'os, et que le rapprochement de ceux-ci dépend uniquement de la précision avec laquelle ont été effectués les perçages.

Divers types d'agrafes chirurgicales ont été proposés pour pallier cet inconvénient .

L'une de ces agrafes chirurgicales est réalisée dans un métal à mémoire de forme, en sorte que lorsqu'elle est froide ses jambes soient parallèles, ce qui permet son introduction dans les perçages, alors que son réchauffement provoque le rapprochement des extrémités des jambes, et donc celui des fragments d'os.

Une telle agrafe demeure d'un coût relativement élevé, et de plus il peut arriver qu'au lieu de se rapprocher, les jambes s'écartent, ce qui est peut avoir pour le patient des conséquences préjudiciables.

D'autres agrafes chirurgicales permettent le rapprochement des jambes, qui demeurent parallèles tandis que la partie transversale se déforme en raccourcissement.

Ainsi on connaît des agrafes dont la partie transversale reliant les deux jambes est constituée de deux branches écartées l'une de l'autre et se rejoignant sur chacune des jambes. De telles agrafes sont notamment décrites dans les documents US 4.444.181, US 4.887.601 et US 5.026.390, qui portent sur des modes de réalisations différentes.

Selon un de ces modes de réalisation, les branches de la partie transversale de ces agrafes sont relativement élastiques et les jambes de l'agrafe sont introduites dans les perçages en exerçant une compression desdites branches l'une vers l'autre, en sorte qu'après relâchement de la pression les branches tendent à reprendre leur forme initiale et opèrent ainsi un rapprochement des jambes.

Ce type d'agrafe présente toutefois l'inconvénient d'un maintien en pression limité du fait qu'il est lié à l'élasticité des branches. L'usage de ce type d'agrafe est particulièrement réservé à la coaptation de petits fragments osseux.

Dans un autre mode de réalisation, les branches de la partie transversale de l'agrafe forment un oeil qui, après introduction des jambes dans les perçages, est ouvert au moyen d'un outil adapté, l'écartement des branches entraînant le raccourcissement de la partie transversale qui provoque le rapprochement des jambes.

Ce type d'agrafe chirurgicale présente l'inconvénient qu'en pratique il est difficile de maîtriser la force à exercer sur l'outil pour déformer l'oeil, du fait notamment de la taille réduite d'une agrafe et donc de sa relative fragilité, en sorte que lorsque le raccourcissement de la partie transversale est trop important, les extrémités libres des jambes, après s'être rapprochées, s'écartent l'une de l'autre.

Ainsi, lorsque la pression exercée sur l'outil dépasse un certain seuil, on observe une ouverture de l'angle que font les jambes avec la partie transversale, ce qui peut favoriser l'expulsion de l'agrafe.

On notera que dans le document US 4.444.181 les branches de la partie transversale sont écrasées de manière à fermer l'oeil, en sorte que l'inconvénient évoqué ci-dessus est peu fréquent, mais que le maintien est moindre du fait de l'amplitude limitée de l'écrasement et donc du rapprochement des jambes.

La présente invention a pour but de proposer une agrafe chirurgicale permettant de remédier à ces divers inconvénients.

L'agrafe chirurgicale objet de la présente invention est du type comportant deux jambes sensiblement parallèles reliées par une partie transversale présentant deux branches et formant un oeil dont la déformation en écartement, dans le sens perpendiculaire à l'axe de ladite partie transversale, permet le rapprochement desdites jambes, et elle se caractérise essentiellement en ce que lesdites branches comportent plusieurs zones de flexion, de manière à permettre la déformation dudit oeil en exerçant une pression, dans le sens axial de ladite partie transversale, sur les extrémités desdites jambes qui émergent de la matière osseuse.

Selon une caractéristique additionnelle du dispositif selon l'invention chacune des branches comporte trois zones de flexion, l'une dans sa région médiane et les deux autres à chacune de ses extrémités, dans les parties de jonction avec les jambes.

Selon une autre caractéristique additionnelle du dispositif selon l'invention, les branches sont en forme de V ouvert.

Selon une autre caractéristique additionnelle du dispositif selon l'invention la zone de flexion médiane d'une branche consiste en une échancrure pratiquée du côté interne.

Selon une autre caractéristique additionnelle du dispositif selon l'invention, les zones de flexion que comporte une branche à chacune de ses extrémités consistent chacune en une échancrure pratiquée du côté externe dans les parties de jonction de la partie transversale.

Après mise en place de l'agrafe chirurgicale selon l'invention, le chirurgien positionne les mors d'un outil adapté, du type pince, sur les extrémités des jambes qui émergent de la matière osseuse, et exerce une pression de manière à ouvrir l'oeil et à rapprocher lesdites jambes. Le fait d'exercer la pression au niveau des jambes permet d'éviter l'ouverture de l'angle qu'elles forment avec la partie transversale.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue en perspective d'une agrafe chirurgicale selon l'invention.
- la figure 2 représente une vue en plan de dessus de la même agrafe chirurgicale, dans deux configurations, avant et pendant une déformation.
- la figure 3 représente une vue schématique en coupe de la coaptation de deux fragments osseux au moyen de la même agrafe.

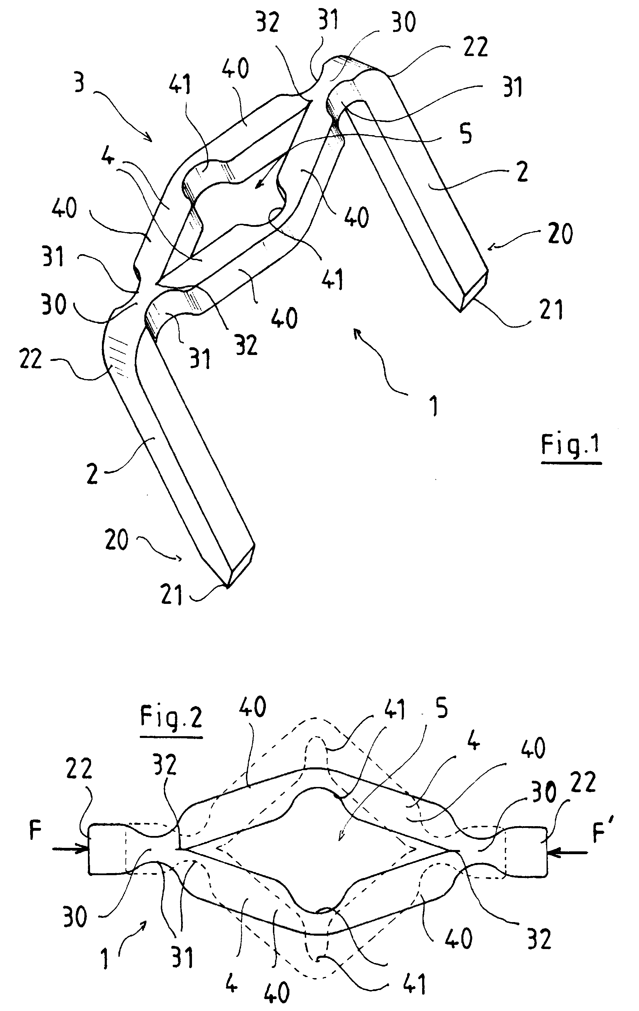

Si on se réfère à la figure 1, on peut voir qu'une agrafe 1 selon l'invention comporte deux jambes 2 reliées par une partie transversale 3.

Les extrémités libres 20 des jambes 2 présentent chacune une pointe 21, tandis que leurs autres extrémités 22 sont jointes par la partie transversale 3 qui est sensiblement perpendiculaire aux jambes 2.

Les jambes 2 sont destinées à être introduites dans des perçages pratiqués dans la matière osseuse de part et d'autre d'un trait de fracture.

La partie transversale 3 comporte deux branches 4 qui présentent chacune deux parties 40 faisant entre elles un angle ouvert, les branches 4 sont ainsi chacune en forme de V.

Les branches 4 se rejoignent à proximité des extrémités 22 des jambes 2 par l'intermédiaire de parties de jonction 30, et forment alors un oeil 5 en forme de losange.

Les branches 4 comportent chacune dans leur région médiane, au niveau de la jonction des parties 40, et du côté intérieur, une échancrure 41 qui crée une zone de moindre résistance, dans le but de faciliter une flexion permettant de fermer l'angle que forment les parties 40 d'une branche 4.

D'autre part, les parties de jonction 30 présentent chacune extérieurement, de part et d'autre, deux échancrures 31, destinées à permettre une flexion en écartement de deux parties 40 de deux branches 4.

D'autre part encore, chacune des parties de jonction 30 présente du côté intérieur une fente 32 pratiquée selon l'axe de la partie transversale 3 et s'étendant partiellement entre les échancrures 31, les fentes 32 étant destinées à faciliter la flexion.

La disposition des échancrures 31 et 41 permet d'orienter la déformation de la partie transversale 3, en sorte que cette déformation soit réalisée dans le plan de la partie transversale 3.

On notera également que les échancrures 31 et 41 représentées sont de profils arrondis, mais peuvent toutefois présenter une autre forme, telle qu'une entaille triangulaire par exemple, la forme arrondie offrant l'avantage de limiter le risque de rupture lors de la déformation.

Si on se réfère maintenant à la figure 2, on peut voir la partie transversale 3 représentée avant déformation en traits pleins, et lors d'une déformation en traits discontinus.

La déformation est réalisée en exerçant une pression, représentée par les flèches F et F', sur les extrémités 22 des jambes 2, en sorte que les fentes 32 s'ouvrent et les échancrures 31 et 41 se referment, et que les branches 4 s'éloignent l'une de l'autre, du moins leurs zones médianes, c'est-à-dire que les parties 40 d'une même branche 4 se rapprochent pour former un angle plus fermé, alors que les parties 40 en vis-à-vis de deux branches 4 s'écartent pour former un angle plus ouvert.

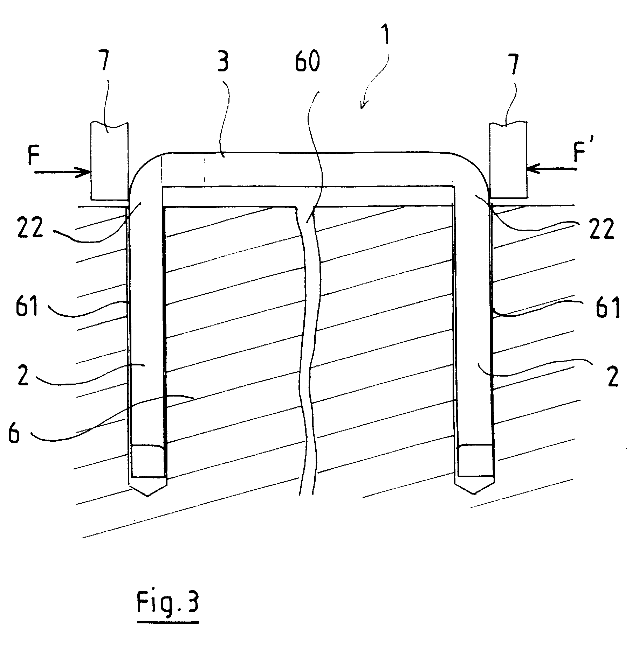

Si on se réfère maintenant à la figure 3 on peut voir de la matière osseuse 6 présentant un trait de fracture 60 qu'il s'agit de traiter au moyen d'une agrafe 1.

En pratique, deux perçages 61 sont pratiqués dans la matière osseuse 6, de part et d'autre du trait de fracture 60, et les jambes 2 de l'agrafe 1 sont introduites dans les perçages 61.

Ensuite, on déforme la partie transversale 3 au moyen d'un outil du type pince, dont seules les extrémités 7 des mors sont représentées sur la figure.

Les mors 7 sont appliqués sur les extrémités 22 des jambes 2, et une pression est exercée dans le sens axial de la partie transversale 3, c'est-à-dire dans le sens des flèches F et F'.

Du fait de la pression axiale, la partie transversale 3 est déformée et les jambes 2 sont rapprochées l'une de l'autre en demeurant parallèles.

On notera de plus que le fait d'agir en pression directement sur les jambes 1 permet de contrôler plus facilement le serrage.

## Revendications

1. Agrafe chirurgicale utilisée dans le domaine de la chirurgie orthopédique, du type comportant deux jambes (1) sensiblement parallèles reliées par une partie transversale (3) présentant deux branches (4) et formant un oeil (5) dont la déformation en écartement, dans le sens perpendiculaire à l'axe de ladite partie transversale (3), permet le rapprochement desdites jambes (1), caractérisée en ce que lesdites branches (4) comportent plusieurs zones de flexion (31, 41) de manière à permettre la déformation dudit oeil (5) en exerçant une pression, dans le sens axial de ladite partie transversale (3), sur les extrémités (22) desdites jambes (2) qui émergent de la matière osseuse (6).

2. Agrafe chirurgicale selon la revendication 1, caractérisée en ce que chacune des branches (2) comporte trois zones de flexion (31, 41), l'une dans sa région médiane, et les deux autres à chacune de ses extrémités, dans les parties de jonction (30) avec les jambes (2).

3. Agrafe chirurgicale selon la revendication 1 ou la revendication 2 caractérisée en ce que les branches (4) sont en forme de V ouvert.

4. Agrafe chirurgicale selon la revendication 2 ou la revendication 3 caractérisée en ce que la zone de flexion médiane d'une branche (4) consiste en une échancrure (41) pratiquée du côté interne.

5. Agrafe chirurgicale selon l'une quelconque des revendications 2 à 4, caractérisée en ce que les zones de flexion que comporte une branche (40) à chacune de ses extrémités consistent chacune en une échancrure (31) pratiquée du côté extérieur dans les parties de jonction (30) de la partie transversale (3).

6. Agrafe chirurgicale selon la revendication 5 caractérisée en ce qu'une fente axiale (32) est pratiquée dans chacune des parties de jonction (30) de la partie transversale (3), ladite fente (32) s'étendant partiellement entre les deux échancrures (31) pratiquées extérieurement dans ladite partie de jonction (30).
